# EUROPÄISCHE PATENTSCHRIFT

(11) **EP 2 125 165 B1**
(45) Veröffentlichungstag und Bekanntmachung des Hinweises auf die Patenterteilung: **27.05.2015**
(21) Anmeldenummer: 08707671.7
(22) Anmeldetag: 12.02.2008
(51) Int. Cl.: B01D 53/18, B01J 19/00, C07D 251/60, C07D 251/62

(54) **VERWENDUNG EINES GASWÄSCHERS IN DER MELAMINPRODUKTION**
USE OF A GAS SCRUBBER IN MELAMINE PRODUCTION
UTILISATION D'UN LAVEUR DE GAZ DANS LE CADRE D'UNE PRODUCTION DE MÉLAMINE

(30) Priorität: 16.02.2007 DE 102007007746
(43) Veröffentlichungstag der Anmeldung: 02.12.2009
(73) Patentinhaber: Air Liquide Global E&C Solutions Germany GmbH, 60439 Frankfurt am Main (DE)
(72) Erfinder: SCHADT, Arne, A-4020 Linz (AT); EBERHARDT, Juergen, 63110 Rodgau (DE); MUELLER-HASKY, Martin, 63150 Heusenstamm (DE)
(86) Internationale Anmeldenummer: PCT/EP2008/001047
(87) Internationale Veröffentlichungsnummer: WO 2008/098732

(56) Entgegenhaltungen:
- WO-A-02/100840
- DE-A1- 1 542 406
- DE-A1- 10 229 101
- DE-A1-102005 023 041
- US-A- 1 964 357
- US-A- 3 700 672
- US-A- 3 746 322
- Week 198604, Derwent Publications Ltd., London, GB; AN 1986-027372 & SU 1 166 811 A (BLYAKHER I G) 15 Juli 1985

## Beschreibung

Die Erfindung betrifft die Verwendung eines Gaswäschers in einer Vorrichtung zum Herstellen von C₃N₃(NH₂)₃ aus CO(NH₂)₂ und ein Verfahren zum Betrieb der Vorrichtung zum Herstellen von C₃N₃(NH₂)₃ aus CO(NH₂)₂.

C₃N₃(NH₂)₃ ist ein durch Trimerisierung von CO(NH₂)₂ gewonnenes wichtiges Zwischenprodukt für die Herstellung von Duroplasten, Leimen, Klebstoffen etc. Die Salze von C₃N₃(NH₂)₃ haben mit anorganischen und organischen Säuren als Flammschutzmittel und als Beschleuniger für Aminoplastharze Bedeutung erlangt. Ausgehend von CO(NH₂)₂ lässt sich C₃N₃(NH₂)₃ sowohl nach dem Hochdruck-Verfahren in Gegenwart eines handelsüblichen Katalysators, wie Aluminiumsilikate, Aluminiumoxid oder Kieselgel bei einem Druck von mindestens 80 bar[a] als auch nach dem Niederdruck-Verfahren in Gegenwart eines der vorgenannten Katalysatoren bei einem Druck von höchstens 10 bar[a] und bei einer Temperatur von 380 bis 410° C in einer Wirbelschicht gemäß der Reaktionsgleichung

6 CO(NH₂)₂ → C₃N₃(NH₂)₃ + 6 NH₃ + 3 CO₂

herstellen, wobei NH₃ oder eine Mischung aus CO₂ und NH₃ als Wirbelgas eingesetzt wird. Da die Reaktion endotherm verläuft, müssen größere Wärmemengen von außen dem Prozess zugeführt werden. Bezogen auf die eingesetzte Menge CO(NH₂)₂ beträgt die Ausbeute an C₃N₃(NH₂)₃ in aller Regel 90 bis 95 %.

In der WO-A-2006/119815 ist ein einstufiges Verfahren zum Herstellen von C₃N₃(NH₂)₃ beschrieben, bei dem geschmolzener CO(NH₂)₂ in einer Wirbelschicht bei einer Temperatur von 395 bis 400° C und nahezu Atmosphärendruck in Gegenwart eines Katalysators zu Synthesegas, enthaltend sublimiertes C₃N₃(NH₂)₃, NH₃ und CO₂ und Spuren von Nebenprodukten, wie die polycyclischen Verbindungen Melam und Melem, umgesetzt wird. Anschließend wird das Synthesegas gekühlt, dann zum Entfernen der Nebenprodukte und der Katalysatorpartikeln gefiltert und danach einem Kristallisator zugeführt, in dem das Synthesegas auf eine Temperatur von 190 bis 210° C gekühlt wird. Durch das Kühlen des Synthesegases wird das sublimierte C₃N₃(NH₂)₃ zu annähernd 98 % resublimiert und die gebildeten C₃N₃(NH₂)₃-Kristalle in einem nachgeschalteten Abscheider von dem Synthesegas getrennt und aus dem Prozess ausgeleitet. Der im wesentlichen CO₂, NH₃, HNCO und eine geringe Menge an sublimiertem C₃N₃(NH₂)₃ enthaltende Synthesegasrest wird dem Kopf eines Gaswäschers aufgegeben und in diesem mit einer eine Temperatur von 130 bis 145° C besitzender, in den Gaswäscher gesprühter CO(NH₂)₂-Schmelze auf eine Temperatur von 190 bis 220° C abgekühlt, so dass das in dem Synthesegasrest enthaltende C₃N₃(NH₂)₃ resublimiert und HNCO in Gegenwart von NH₃ zu CO(NH₂)₂ umgesetzt wird. Das resublimierte C₃N₃(NH₂)₃, die gewaschenen Gase NH₃ und CO₂ und die CO(NH₂)₂ werden aus dem Gaswäscher getrennt ausgeleitet und die CO(NH₂)₂-Schmelze in den Prozess rückgeführt. Ein Nachteil dieses Verfahrens besteht darin, dass sich im Oberteil des Gaswäschers auf grund der relativ geringen Durchmischung von CO(NH₂)₂ und Synthesegasrest resublimiertes C₃N₃(NH₂)₃ an den Einbauten niederschlägt und von dort nur unter Anwendung mechanischer Hilfsmittel entfernt werden kann. Darüber hinaus besteht die Tendenz, dass sich Umsetzungsprodukte des Harnstoffs, wie Cyanursäure, Biruet, Triuret etc. an schwer zugänglichen Stellen des Gaswäschers ablagern.

Als grundsätzlich für diese Anwendung geeignete Wäscherbauarten werden von US 3,700,672 als Beispiele der Sprühturm oder ein mit Lochplatten, Glockenböden oder Füllkörpern ausgestatteter Waschturm oder ein Venturi-Jet-Wäscher genannt.

Es ist die Aufgabe der vorliegenden Erfindung einen Gaswäscher bereitzustellen, in dem der sublimiertes C₃N₃(NH₂)₃ enthaltende Synthesegasrest mit einem CO(NH₂)₂- Schmelzestrom bereits zum Anfang des Kontakts zwischen den beiden Strömen innig mischbar ist.

Gelöst ist diese Aufgabe durch wenigstens einen im Oberteil des Gaswäschers angeordneten Lochboden mit mehreren jeweils von einem Staurand umgebenen Löchern, oberhalb des Lochbodens angeordneten Zuläufen für den Gasstrom und den Flüssigkeits- bzw. Schmelzestrom, einem in der Kontaktstrecke, in der der Gasstrom mit dem Flüssigkeits- bzw. Schmelzestrom in Berührung kommt, unterhalb des Lochbodens angeordneten Wärmeübertrager und im Unterteil des Gaswäschers getrennt angeordnete Abläufe für die gereinigten Gase und den Flüssigkeits- bzw. Schmelzestrom.

Die über entsprechende Zuläufe auf die Lochplatte geleiteten Flüssigkeits- bzw. Schmelzestrom und Gasstrom werden zunächst durch die die Löcher umgebenden Stauränder am unmittelbaren Abströmen über die Löcher der Lochplatte gehindert. Durch den sich bildenden Stau beginnen sich Flüssigkeits- bzw. Schmelzestrom und Gasstrom zu mischen. Die Mischwirkung wird dadurch verbessert, dass die Zuläufe für den Flüssigkeits- bzw. Schmelzestrom und den Gasstrom gegeneinander gerichtet in dem Gaswäscher angeordnet sind. Sobald das Gemisch den Scheitel der Stauränder übersteigt, wird durch das über die Stauränder und die Löcher abfallende Gemisch ein Unterdruck und eine Erhöhung der Fließgeschwindigkeit erzeugt mit der Folge, dass eine verstärkte Berührung von Gas und Flüssigkeit bzw. Schmelze stattfindet.

Eine sehr intensive und innige Berührung von Gas und Flüssigkeit bzw. Schmelze wird erreicht, indem der die Löcher umgebende Staurand die Form eines Konus oder des oberen Abschnitts eines Rotationsparaboloids besitzt und auf der Unterseite des Lochbodens jeweils ein die Löcher umgebender Rand in Form eines Gegenkonus oder Gegenabschnitts des Rotationsparaboloids angebracht ist. Durch die beiden gegeneinander gerichteten Konen bzw. Abschnitte des Rotationsparaboloids, die sich beim engsten Querschnitt, d.h. in den Löchern der Lochplatte vereinen, werden ein größerer Unterdruck und eine Erhöhung der Fließgeschwindigkeit des aus Gas und Flüssigkeit bzw. Schmelze gebildeten Gemisches an der engsten Stelle erzielt. An der engsten Stelle ist der Staudruck maximal und der statische Druck minimal. Die Strömungsgeschwindigkeit des Gemisches steigt beim Durchströmen des Zulaufkonus bzw. des Zulaufabschnitts des Rotationsparaboloids an, gleichzeitig sinkt der Druck im Ablaufkonus bzw. im Ablaufabschnitt des Rotationsparaboloids. Durch die entstehenden Strömungsverhältnis wird ein homogenes Gemisch aus Gas und Flüssigkeit bzw. Schmelze erzeugt.

Die erfindungsgemäße Vorrichtung besteht aus einem Wirbelschichtreaktor zum Umsetzen von CO(NH₂)₂ in Gegenwart eines handelsüblichen Katalysators bei einer Temperatur von 380 bis 420° C und einem Druck von höchstens 10 bar[a] zu einem sublimiertes C₃H₃(NH₂)₃ sowie NH₃, CO₂ und HNCO enthaltenden Synthesegas, einem Wärmeübertrager zum Abkühlen des Synthesegases auf eine Temperatur von 210 bis 300° C, einem Gasfilter zum Entfernen von in dem Synthesegas enthaltenen staubförmigen Katalysatorpartikeln und Nebenprodukten der katalytischen Umsetzung, wie Melem und Melam, einem Kristallisator für die Resublimierung des sublimierten C₃N₃(NH₂)₃ unter Abkühlung des Synthesegases auf eine Temperatur von 180 bis 210° C, einem Abscheider zum Entfernen der durch die Resublimierung gebildeten C₃N₃(CH₂)₃-Kristalle aus dem Synthesegas, einem Gaswäscher zum Entfernen der in dem Synthesegasrest enthaltenen Gaskomponenten CO₂, NH₃, HNCO sowie ggf. von in Spuren vorhandenen sublimierten C₃N₃(NH₂)₃ und getrennte Abläufe für das gereinigte NH₃ und CO₂ enthaltende Gasgemisch sowie die CO(NH₂)₂-Schmelze aus dem Gaswäscher. Die in den Gaswäscher eingeleitete CO(NH₂)₂-Schmelze und der zugeführte Synthesegasrest werden durch den gemäß der Erfindung gestalteten Gaswäscher so intensiv und innig miteinander gemischt, dass sich weder in dem Synthesegasrest ggf. in Spuren vorhandenes sublimiertes C₃N₃(NH₂)₃ in resublimierter Form noch Umsetzungsprodukte des CO(NH₂)₂ an Einbauten des Gaswäschers oder an unzugänglichen Stellen des Gaswäschers niederschlagen. Die eine Temperatur von 120 bis 150° C, vorzugsweise 135 bis 145° C besitzende CO(NH₂)₂-Schmelze und der eine Temperatur von 190 bis 210° C aufweisende Synthesegasrest fließen in gegeneinander gerichteten Strömen auf die Lochplatte und vermischen sich auf der Lochplatte zu einem heterogenen Gemisch, das mit einer Mischtemperatur von 155 bis 175° C durch die Löcher der Lochplatte in den darunter liegenden Abschnitt des Gaswäschers unter Bildung eines homogenen Gemisches aus CO(NH₂)₂-Schmelze und Synthesegasrest abfließt. Das homogene Gemisch wird auf dem Kontaktweg unterhalb der Lochplatte auf eine Temperatur von 130 bis 150° C abgekühlt.

Die Erfindung wird durch ein Ausführungsbeispiel und ein in der Zeichnung wiedergegebenes Grundfließschema sowie einen schematischen Längsschnitt eines Gaswäschers näher erläutert. Es zeigen:
- Fig. 1: ein Grundfließschema eines Niederdruck-Verfahrens zum Herstellen von C₃N₃(NH₂)₃
- Fig. 2: einen schematischen Längsschnitt durch einen Gaswäscher für die Verwendung in der Vorrichtung zum Herstellen von C₃N₃(NH₂)₃ nach Fig. 1

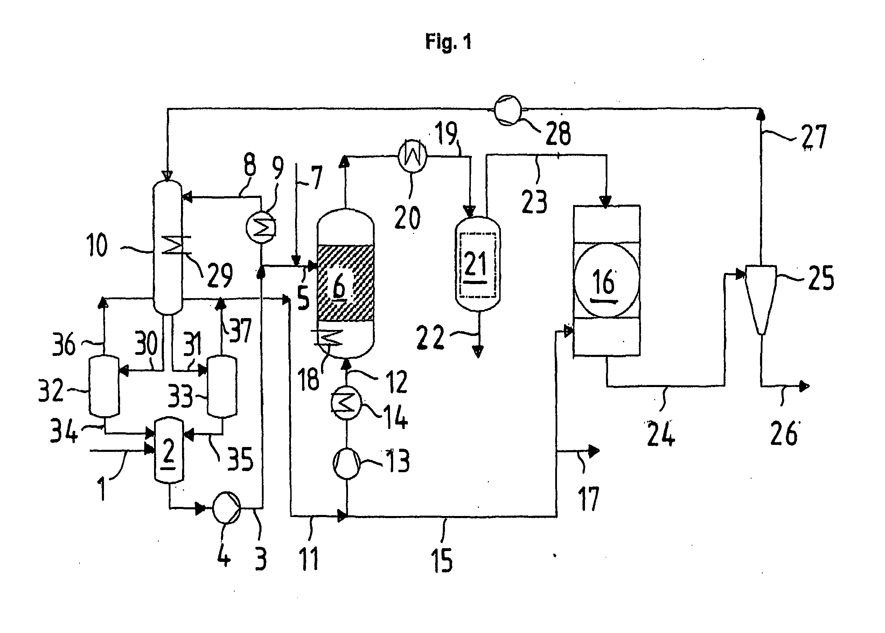

Gemäß Fig. 1 wird aus dem über Leitung (1) mit CO(NH₂)₂-Schmelze befüllten Vorratsbehälter (2) CO(NH₂)₂-Schmelze mit einer Temperatur von 135 bis 145° C abgezogen und durch Leitung (3) über die Pumpe (4) und durch Leitung (5) zum einen Teil dem Wirbelschichtreaktor (6), in dem als Katalysator, beispielweise Al₂O₃, eingesetzt ist, aufgegeben. Unmittelbar vor dem Eintritt der CO(NH₂)₂-Schmelze in den Wirbelschichtreaktor (6) wird in die in Leitung (5) strömende CO(NH₂)₂-Schmelze durch Leitung (7) zugeführtes NH₃ eingespeist, um ein Versprühen der CO(NH₂)₂-Schmelze unmittelbar nach deren Eintritt in den Wirbelschichtreaktor (6) mit der Folge eines sofortigen Verdampfens der CO(NH₂)₂-Schmelze zu erreichen. Der andere Teil der als Waschmittel dienenden CO(NH₂)₂-Schmelze strömt im Kreislauf durch Leitung (8) über den Wärmeübertrager (9), in dem die Schmelze auf eine Temperatur von 120 bis 130° C gekühlt wird, über den im Kopf befindlichen Zulauf in den Gaswäscher (10). Aus dem Unterteil des Gaswäschers (10) wird über Leitung (11) ein NH₃ und CO₂ enthaltendes eine Temperatur von 120 bis 150° C besitzendes Gasgemisch abgezogen und zu einem Teil durch Leitung (12) über den Kompressor (13) und den für die Vorwärmung auf eine Temperatur von 380 bis 420° C dienenden Wärmeübertrager (14) als Wirbelgas in den unteren Teil des Wirbelschichtreaktor (6) geleitet und zum andern Teil durch Leitung (15) als Kühlgas dem Kristallisator (16) aufgegeben. Dabei besteht die Möglichkeit aus Leitung (15) durch Leitung (17) NH₂ und CO₂ enthaltendes Gasgemisch aus dem Prozess abzuziehen. Im unteren Abschnitt des Wirbelschichtreaktors (6) befindet sich ein Wärmeübertrager (18), mit dem für die katalytische Umsetzung zusätzlich erforderliche Wärme in die Wirbelschicht eingebracht werden kann. Die Temperatur der Wirbelschicht beträgt 380 bis 420° C. Das infolge der katalytischen Umsetzung im Wirbelschichtreaktor (6) sublimierte C₃N₃(NH₂)₃ strömt zusammen mit dem Wirbelgas und dem bei der katalytischen Umsetzung neu gebildeten NH₃ und CO₂ durch Leitung (19) über den Wärmeübertrager (20), in dem das Gasgemisch auf eine Temperatur von 210 bis 300° C gekühlt wird, so dass die bei der katalytischen Umsetzung im Wirbelschichtreaktor (6) entstandenen Nebenprodukte, wie Melem und Melam, kondensieren. Die Nebenprodukte und die in dem Gasgemisch enthaltenen mitgerissenen staubförmigen Katalysatorpartikel werden in dem nachgeschalteten Gasfilter (21) abgetrennt und durch Leitung (22) aus dem Prozess geleitet. Durch Leitung (23) wird das von Nebenprodukten der katalytischen Umsetzung und mitgerissenen staubförmigen Katalysatorpartikeln gereinigte Gasgemisch in den Kristallisator (16), in dem das Gasgemisch mittels dem durch Leitung (15) zugeführten NH₃ und CO₂ enthaltenden Gasgemisch auf eine Temperatur von 150 bis 250° C, vorzugsweise 190 bis 210° C, abgekühlt wird, geleitet. Das als Folge der Kühlung in Kristallform resublimierte C₃N₃(NH₂)₃ wird zusammen mit NH₃ und CO₂ durch Leitung (24) einem Abscheider (25), vorzugsweise einem Gaszyklon, aufgegeben, in dem die resublimierten C₃N₃(NH₂)₃-Kristalle abgeschieden und durch Leitung (26) aus dem Prozess geleitet wird. Der Synthesegasrest, der NH₃, CO₂, HNCO sowie ggf. Spuren von sublimiertem C₃N₃(NH₂)₃ enthält, wird aus dem Abscheider (25) durch Leitung (27) über den Kompressor (28) dem im Kopf des Gaswäschers (10) angebrachten Zulauf aufgegeben. In dem Gaswäscher (10) wird der Synthesegasrest mit eine Temperatur von 120 bis 130° CO(NH₂)₂-Schmelze gewaschen und gleichzeitig auf eine Temperatur von 130 bis 150° C gekühlt, indem die CO(NH₂)₂-Schmelze über einen im Mittelabschnitt des Gaswäschers (10) angeordneten Wärmeübertrager geleitet wird. Die CO(NH₂)₂-Schmelze sammelt sich im Sumpf des Gaswäschers (10) und wird über Leitungen (30, 31) jeweils einem Tropfenabscheider (32, 33) zugeführt, in dem die CO(NH₂)₂-Schmelze und das abgeschiedene NH₃ und CO₂ enthaltende Gasgemisch voneinander getrennt werden. Die CO(NH₂)₂-Schmelze wird aus den Tropfenabscheidern (32,33) durch Leitungen (34, 35) in den Vorratsbehälter (2) für die CO(NH₂)₂-Schmelze abgegeben. Das in den Tropfenabscheidern (32, 33) abgetrennte NH₃ und CO₂ enthaltende Gasgemisch wird durch Leitungen (36, 37) in Leitung (11) eingespeist und teilweise als Wirbelgas durch Leitung (12) dem Wirbelschichtreaktor (6) und teilweise durch Leitung (15) als Kühlgas dem Kristallisator (16) zugeführt. Die Anordnung eines Wärmeübertragers in Leitung (8) ist nicht zwingend erforderlich, so dass die CO(NH₂)₂-Schmelze aus dem Vorratsbehälter (2) ohne Zwischenkühlung dem Gaswäscher zugeführt werden kann.

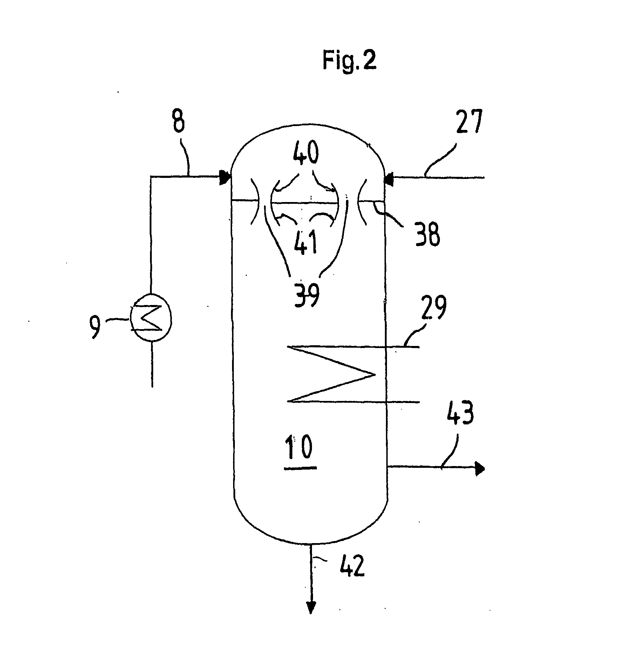

Bei dem in Fig. 2 im Längsschnitt schematisch dargestellten Gaswäscher (10) ist im Oberteil ein Lochboden (38) mit mehreren Löchern (39) angeordnet, die auf der Oberseite des Lochbodens (38) mit jeweils von einem die Form des oberen Abschnitts eines Rotationsparaboloids aufweisenden Staurands (40) und auf der Unterseite von einem die Form des Gegenabschnitts des Rotationsparaboloids aufweisenden Rands (41) umgeben sind. Oberhalb des Lochbodens (38) sind in der Wand des Gaswäschers (10) einander gegenüberliegend der Zulauf der Leitung (27) für den Synthesegasrest und der Zulauf der Leitung (8) für die CO(NH₂)₂-Schmelze angebracht. Im Sumpf des Gaswäschers (10) sammelt sich die CO(NH₂)₂-Schmelze, wird von dort über Leitung (42) abgezogen und in den Prozess rückgeführt. Das gereinigte NH₃ und CO₂ enthaltende Gasgemisch verlässt den Gaswäscher (10) über einen oberhalb des Sumpfs angeordnet Ablauf (43) und wird teilweise als Wirbelgas durch Leitung (12) in den Wirbelschichtreaktor (6) und teilweise zur Kühlung durch Leitung (15) in den Kristallisator (16) geleitet.

### Ausführungsbeispiel:

Aus dem Vorratsbehälter (2) für die CO(CH₂)₂-Schmelze werden 22 t/h CO(CH₂)₂-Schmelze mit einer Temperatur von 128° C durch Leitung (3) über die Pumpe (4) und Leitung (5) in den Wirbelschichtreaktor (6) geleitet, wobei in die in Leitung (5) strömende CO(NH₂)₂-Schmelze über Leitung (7) zugeführtes NH₃ eingespeist wird. Die Umsetzung des dampfförmigen CO(NH₂)₂ zu C₃N₃(NH₂)₃ erfolgt in Gegenwart Al₂O₃ bei einer Temperatur von 400° C und einem Druck von 3 bar(a). Das durch Leitung (11) zuströmende NH₃ und CO₂ enthaltende Gasgemisch wird zu einem Teil über den Kompressor (13) und den der Vorwärmung des Gasgemisches dienenden Wärmeübertrager (14) durch Leitung (12) als Wirbelgas in den Wirbelschichtreaktor (6) eingeleitet. Die für die katalytische Umsetzung zusätzlich benötigte Wärme wird über den Wärmeübertrager (18) in die Wirbelschicht eingetragen. Das in dem Wirbelschichtreaktor (6) gebildete sublimiertes C₃N₃(NH₂)₃, NH₃, CO₂, HNCO, Nebenprodukte der katalytischen Umsetzung, staubförmige Katalysatorpartikel und staubförmige inerte Stoffpartikel enthaltende Synthesegas verlässt den Wirbelschichtreaktor (6) über Leitung (19) und wird in dem Wärmeübertrager (20) auf eine Temperatur von 255° C gekühlt. In dem nachfolgenden Gasfilter (21) werden die bei der Kühlung kondensierten Nebenprodukte der katalytischen Umsetzung sowie staubförmigen Katalysatorpartikel und staubförmige inerte Stoffpartikel aus dem Synthesegas entfernt und über Leitung (22) aus dem Gasfilter (21) geleitet. Das Synthesegas fließt über Leitung (23) in den Kristallisator (16) und wird in diesem mit dem eine Temperatur von 125° C aufweisendem NH₃ und CO₂ enthaltenden Gasgemisch gemischt und dabei auf eine Temperatur von 200° C gekühlt, so dass C₃N₃(NH₂)₃ in Kristallform resublimiert. Die C₃N₃(NH₂)₃-Kristalle werden zusammen mit NH₃, CO₂ und HNCO über Leitung (24) einem Gaszyklon (25) aufgegeben, in dem die C₃N₃(NH₂)₃-Kristalle von dem Synthesegas abgetrennt und über Leitung (26) aus dem Prozess geleitet werden. Der NH₃, CO₂, HNCO und ggf. Spuren von sublimiertem C₃N₃(CH₂)₃ enthaltende eine Temperatur von 206° C besitzende Synthesegasrest wird über Leitung (27) und den Kompressor (28) dem Kopf des Gaswäschers (10) aufgegeben und in diesem mit eine Temperatur von 128° C besitzender über Leitung (8) dem Kopf des Gaswäschers (10) auf gleicher Höhe wie der Synthesegasrest in einer Menge von 1460 t/h zugeführter CO(NH₂)₂-Schmelze gewaschen. Die sich Im Sumpf des Gaswäschers (10) sammelnde CO(NH₂)₂-Schmelze wird nach der Abtrennung der Komponenten NH₃ und CO₂ in den Vorratsbehälter (2) geleitet und von diesem aus in den Prozess rückgeführt.

## Patentansprüche

1. Verwendung eines Gaswäschers (10)für eine Vorrichtung zum Herstellen von C₃N₃(NH₂)₃ aus CO(NH₂)₂, bestehend aus einem Wirbelschichtreaktor (6) zum Umsetzen von CO(NH₂)₂ in Gegenwart eines Katalysators bei einer Temperatur von 380 bis 420° C und einem Druck von höchstens 10 bar[a] zu einem sublimiertes C₃H₃(NH₂)₃ enthaltenden Synthesegas, einem Wärmeübertrager (20) zum Abkühlen des Synthesegases auf eine Temperatur von 210 bis 300° C, einem Gasfilter (21) zum Entfernen von im Synthesegas enthaltenen Nebenprodukten der katalytischen Umsetzung, wie Melem und Melam, staubförmigen Katalysatorpartikeln und staubförmigen inerten Stoffpartikeln, einem Kristallisator (16) für die Resublimierung des sublimierten C₃N₃(CH₂)₃ unter Abkühlung des Synthesegases auf eine Temperatur von 150 bis 250° C, vorzugsweise 190 bis 210° C, einem Abscheider (25) zum Entfernen der resublimierten C₃N₃(NH₂)₃-Kristalle aus dem Synthesegas und dem Gaswäscher (10) zum Entfernen der in dem Synthesegasrest enthaltenen Komponenten CO₂, NH₃, HNCO und ggf. der Spuren von sublimiertem C₃N₃(NH₂)₃ mittels eine niedrigere Temperatur als der Synthesegasrest aufweisender CO(NH₂)₂-Schmelze, **dadurch gekennzeichnet, dass** der Gaswäscher (10) im Oberteil wenigstens einen Lochboden (38) mit mehreren, jeweils von einem Staurand (40) umgebenen Löchern (39) aufweist, wobei der Staurand (40) die Form eines Konus oder des oberen Abschnitts eines Rotationshyperboloids besitzt und auf der Unterseite des Lochbodens (38) die Löcher von einem Rand in Form eines Gegenkonus oder eines Gegenabschnitts des Rotationshyperboloids umgeben sind und wobei der Gasstromwäscher (10) oberhalb des Lochbodens angeordnete Zuläufe (8, 27) für den Gasstrom und den Flüssigkeits- bzw. Schmelzestrom und einen in der Kontaktstrecke unterhalb des Lochbodens befindlichen Wärmeübertrager (29) und im Unterteil angeordnete Abläufe (42, 43) für den gereinigten Gasstrom und den Flüssigkeits- bzw. Schmelzestrom aufweist.

2. Verfahren zum Betrieb der Vorrichtung nach Anspruch 1, **dadurch gekennzeichnet, dass** die eine Temperatur von 120 bis 150° C, vorzugsweise 135 bis 145° C besitzende CO(NH₂)₂-Schmelze und der eine Temperatur von 190 bis 210° C besitzende Synthesegasrest in gegeneinander gerichteten Strömen auf die Lochplatte des Gaswäschers fließen, das über die Löcher der Lochplatte abfließende eine Temperatur von 155 bis 175° C besitzende Gemisch aus CO(NH₂)₂-Schmelze und Synthesegasrest unterhalb der Lochplatte auf eine Temperatur von 130 bis 150°C abgekühlt wird und das gereinigte NH₃ und CO₂ enthaltende Gasgemisch und die CO(NH₂)₂-Schmelze getrennt voneinander aus dem Gaswäscher geleitet werden.

## Claims

1. Use of a gas scrubber (10) for an apparatus for producing C₃N₃(NH₂)₃ from CO(NH₂)₂, comprising a fluidized-bed reactor (6) for converting CO(NH₂)₂ in the presence of a catalyst at a temperature of 380 to 420 °C and a pressure of not more than 10 bar[a] to a synthesis gas containing sublimed C₃H₃(NH₂)₃, a heat exchanger (20) for cooling the synthesis gas to a temperature of 210 to 300 °C, a gas filter (21) for removing by-products of the catalytic conversion contained in the synthesis gas, such as melem and melam, dust-like catalyst particles and dust-like inert material particles, a crystallizer (16) for the resublimation of the sublimed C₃N₃(CH₂)₃ by cooling the synthesis gas to a temperature of 150 to 250 °C, preferably 190 to 210 °C, a separator (25) for removing the resublimed C₃N₃(NH₂)₃ crystals from the synthesis gas and the gas scrubber (10) for removing the components CO₂, NH₃, HNCO and possibly traces of sublimed C₃N₃(NH₂)₃ contained in the synthesis gas residue by means of molten CO(NH₂)₂ having a lower temperature than the synthesis gas residue, **characterized in that** in its upper part the gas scrubber (10) includes at least one perforated tray (38) with a plurality of holes (39) each surrounded by a retaining edge (40), wherein the retaining edge (40) has the shape of a cone or the upper portion of a rotational hyperboloid and on the bottom side of the perforated tray (38) the holes are surrounded by an edge in the form of a counter-cone or a counter-portion of the rotational hyperboloid, and wherein the gas scrubber (10) includes inlets (8, 27) for the gas stream and the liquid or melt stream which are arranged above the perforated tray, and a heat exchanger (29) present in the contact path below the perforated tray as well as outlets (42, 43) for the purified gas stream and the liquid or melt stream which are arranged in the lower part.

2. A method for operating the apparatus according to claim 1, **characterized in that** the molten CO(NH₂)₂ having a temperature of 120 to 150 °C, preferably 135 to 145 °C and the synthesis gas residue having a temperature of 190 to 210 °C flow onto the perforated plate of the gas scrubber in streams directed against each other, the mixture of molten CO(NH₂)₂ and synthesis gas residue, which flows off via the holes of the perforated plate and has a temperature of 155 to 175 °C is cooled to a temperature of 130 to 150 °C below the perforated plate, and the purified gas mixture containing NH₃ and CO₂ and the molten CO(NH₂)₂ are separately discharged from the gas scrubber.

## Revendications

1. Utilisation d'un laveur (10) de gaz pour un dispositif de production de C₃N₃(NH₂)₃ à partir de CO(NH₂)₂, constitué d'un réacteur (6) à lit fluidisé pour la conversion de CO(NH₂)₂ en présence d'un catalyseur à une température de 380 et 420°C sous une pression d'au plus 10 bar [a] en un gaz de synthèse contenant du C₃H₃(NH₂)₃ sublimé, d'un échangeur de chaleur (20) pour refroidir le gaz de synthèse à une température de 210 à 300°C, d'un filtre (21) de gaz pour éliminer des sous-produits de la conversion catalytique contenus dans le gaz de synthèse, comme du mélème et du mélame, des particules de catalyseur sous forme de poussière et des particules de substances inertes sous forme de poussière, d'un cristalliseur (16) pour la resublimation du C₃N₃(CH₂)₃ sublimé avec refroidissement du gaz de synthèse à une température de 150 à 250°C de préférence de 190 à 210°C, d'un séparateur (25) pour retirer les cristaux de C₃N₃(NH₂)₃ resublimés du gaz de synthèse et du laveur (10) à gaz pour retirer les constituants CO₂, NH₃, HNCO et, le cas échéant, les traces de C₃N₃(NH₂)₃ sublimé contenus dans le restant du gaz de synthèse au moyen d'une température plus basse que le restant du gaz de synthèse comportant du CO(NH₂)₂ fondu, **caractérisée en ce que** le laveur (10) de gaz a, dans la partie supérieure, au moins un plateau (38) à trous, ayant plusieurs trous (39) entourés respectivement d'une chicane (40), la chicane (40) ayant la forme d'un cône ou de la partie supérieure d'un hyper boloïde de révolution et, du côté inférieur du plateau (38) à trous, les trous sont entourés par un bord sous la forme d'un contre cône ou d'une contre partie de l'hyperboloïde de révolution et dans lequel le laveur (10) de courant gazeux a, disposé au-dessus du plateau à trous, des entrées (8, 27) pour le courant gazeux et le courant de liquide et de matière fondue et un échangeur de chaleur (29) se trouvant dans la section de contact en dessous du plateau à trous et, disposées dans la partie inférieure, des sorties (42, 43) pour le courant gazeux épuré et pour le courant de liquide et de matière fondue.

2. Procédé pour faire fonctionner le dispositif suivant la revendication 1, **caractérisé en ce que** le CO(NH₂)₂ fondu ayant une température de 120 à 150°C, de préférence de 135 à 145°C, et le restant de gaz de synthèse, ayant une température de 190 à 210°C, passent en courants de sens contraire sur le plateau à trous du laveur de gaz, le mélange, composé de CO(NH₂)₂ fondu et du restant de gaz de synthèse, ayant une température de 155 à 175°C et sortant par les trous du plateau à trous est refroidi en dessous du plateau à trous à une température de 130 à 150°C et le mélange gazeux épuré contenant du NH₃ et du CO₂ et le CO(NH₂)₂ fondu sortent séparément l'un de l'autre du laveur de gaz.
